# EUROPEAN PATENT APPLICATION

(11) **EP 2 559 999 A2**
(43) Date of publication of application: **20.02.2013**
(21) Application number: 11769088.3
(22) Date of filing: 14.04.2011
(51) Int. Cl.: G01N 33/487, G01N 35/08, A61J 1/05

(54) **MICROFLUIDIC DEVICE**

(30) Priority: 15.04.2010 KR 20100035010; 15.04.2010 KR 20100035007
(71) Applicant: Cytogen Co. Ltd., Gyeonggi-do 462-901 (KR)
(72) Inventor: JEON, Byung Hee, Seongnam-si Gyeonggi-do 139-749 (KR)
(74) Representative: Beier, Ralph
(86) International application number: PCT/KR2011/002652
(87) International publication number: WO 2011/129622

(57) **Abstract**

The present invention discloses a micro-fluidic device for separating targets from a sample. The micro-fluidic device includes a filter cabinet, a first filtering unit and a second filtering unit. The filter cabinet includes a first path and a second path branched from the first path. The first path and the second path are formed within the filter cabinet so that a sample containing different kinds of targets can flow through the first path and the second path. The filter cabinet further includes a introduction hole formed in an upper portion of the filter cabinet to supply the sample into the first path and a discharge hole formed in a lower portion of the filter cabinet to discharge the sample from the first path and the second path. The first filtering unit is installed in an upstream portion of the first path to filter the different kinds of targets from the sample, the first filtering unit configured to guide the different kinds of targets toward the second path. The second filtering unit is installed in the second path to receive the different kinds of targets from the first filtering unit and to filter the different kinds of targets on a size-by-size basis.

## Description

### Field of the Invention

The present invention relates to a micro-fluidic device and, more particularly, to a micro-fluidic device for separating targets from a sample.

### Background of the Invention

In recent years, regulations are tightened on a biological test and a clinical test conducted for the sake of treatment of human diseases. As an alternative for the biological test and the clinical test, research and development have been extensively made on the collection of live cells from the human blood. The collection of cells is conducted by different kinds of cell collecting devices such as a micro-fluidic device, a CTC (Circulating Tumor Cell) chip, a filter, and so forth.

U.S. Patent Publication No. 2007/0259424A1 discloses a micro-fluidic device. The micro-fluidic device disclosed in this patent document includes a top layer, a bottom layer and a plurality of obstacles. Binding moieties, e.g., antibodies, charged polymers, or molecules coupled with cells are coated on the surfaces of the obstacles. The obstacles include micro-posts extending in a height direction from the surface of the top layer or the bottom layer. A sample, e.g., the blood, is admitted through an inlet of the top layer to flow along channels and is then discharged through an outlet of the top layer. The cells contained in the blood are captured by the binding moieties.

### Summary of the Invention

### Technical Problems

However, the micro-fluidic device stated above suffers from a problem in that the capture rate of targets is very low. This is because the targets are captured by merely bonding the targets to the binding moieties. Moreover, the micro-fluidic device has a difficulty in collecting the targets captured by the binding moieties. The micro-fluidic device is not suitable for use in separating the targets from a large quantity of blood and in testing and analyzing the targets thus separated.

In view of the problems noted above, it is an object of the present invention to provide a micro-fluidic device capable of efficiently separating targets contained in a sample.

Another object of the present invention is to provide a micro-fluidic device capable of easily separating targets from a sample depending on the size of the targets.

A further object of the present invention is to provide a micro-fluidic device capable of removing non-targets through pre-treatment of non-targets and targets and then efficiently separating the pre-treated targets through post-treatment.

### Means for Solving the Problems

In order to achieve these objects, the present invention provides a micro-fluidic device, including: a filter cabinet including a first path and a second path branched from the first path, the first path and the second path formed within the filter cabinet so that a sample containing different kinds of targets can flow through the first path and the second path, the filter cabinet further including a introduction hole formed in an upper portion of the filter cabinet to supply the sample into the first path and a discharge hole formed in a lower portion of the filter cabinet to discharge the sample from the first path and the second path; a first filtering unit installed in an upstream portion of the first path to filter the different kinds of targets from the sample, the first filtering unit configured to guide the different kinds of targets toward the second path; and a second filtering unit installed in the second path to receive the different kinds of targets from the first filtering unit and to filter the different kinds of targets on a size-by-size basis.

### Effect of the Invention

The micro-fluidic device according to the present invention can efficiently filter and separate different kinds of targets contained in a sample on a size-by-size basis. In addition, non-targets are removed through pre-treatment of non-targets and targets and then the pre-treated targets are separated through post-treatment. Accordingly, the micro-fluidic device is very useful in separating and collecting cells from the human blood and so forth.

### Brief Description of the Drawings

Fig. 1 is a perspective view showing the configuration of a micro-fluidic device according to the present invention.
Fig. 2 is a perspective view showing a body of a filter cabinet of the micro-fluidic device according to the present invention, with a cover removed for clarity.
Fig. 3 is a section view showing the configuration of the micro-fluidic device according to the present invention.
Fig. 4 is an exploded perspective view showing the configuration of a first filtering unit of the micro-fluidic device according to the present invention.
Fig. 5 is an exploded perspective view showing the configuration of a second filtering unit of the micro-fluidic device according to the present invention.
Fig. 6 is a perspective view showing the configuration of a mesh filter employed in the second filtering unit of the micro-fluidic device according to the present invention.
Fig. 7 is a partially cutaway perspective view showing the configuration of the mesh filter employed in the second filtering unit of the micro-fluidic device according to the present invention.
Fig. 8 is a section view for explaining a process in which non-targets are filtered by the first filtering unit of the micro-fluidic device according to the present invention.
Fig. 9 is a section view for explaining a process in which targets are filtered by the second filtering unit of the micro-fluidic device according to the present invention.
Fig. 10 is a perspective view showing a modified example of the mesh filter employed in the micro-fluidic device according to the present invention.
Fig. 11 is a perspective view showing another modified example of the mesh filter employed in the micro-fluidic device according to the present invention.
Fig. 12 is a perspective view showing a further modified example of the mesh filter employed in the micro-fluidic device according to the present invention.
Fig. 13 is a section view showing a micro-fluidic device according to another embodiment of the present invention.
Fig. 14 is an exploded perspective view showing the configuration of a dispersing unit employed in the micro-fluidic device according to another embodiment of the present invention.
Fig. 15 is a section view for explaining a process in which targets are filtered by the dispersing unit and the second filtering unit of the micro-fluidic device according to another embodiment of the present invention.

### Detailed Description of the Preferred Embodiments

Other objects, specific advantages and novel features of the present invention will become apparent from the following description of preferred embodiments made in conjunction with the accompanying drawings.

Certain preferred embodiments of a micro-fluidic device according to the present invention will now be described in detail with reference to the accompanying drawings.

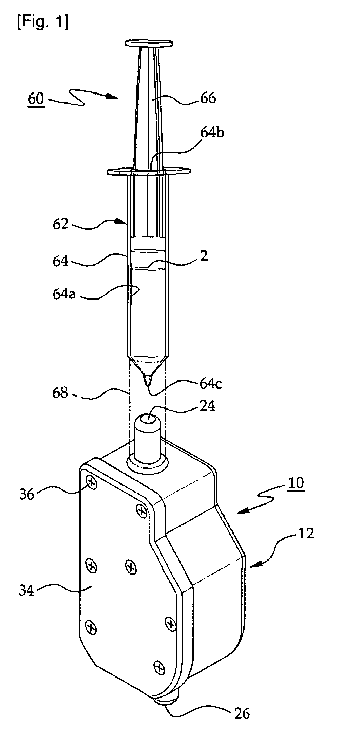

Referring first to Figs. 1 through 3, a micro-fluidic device according to the present invention primarily filters a plurality of non-targets 4 and different kinds of targets 6 (6a, 6b and 6c) contained in a sample 2 to separate the targets 6. Thereafter, the micro-fluidic device secondarily filters and separates the targets 6 on a size-by-size basis.

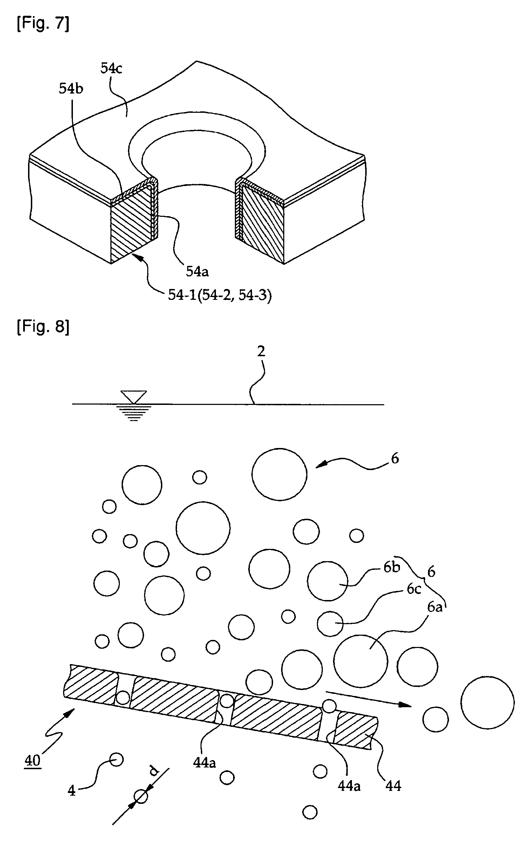

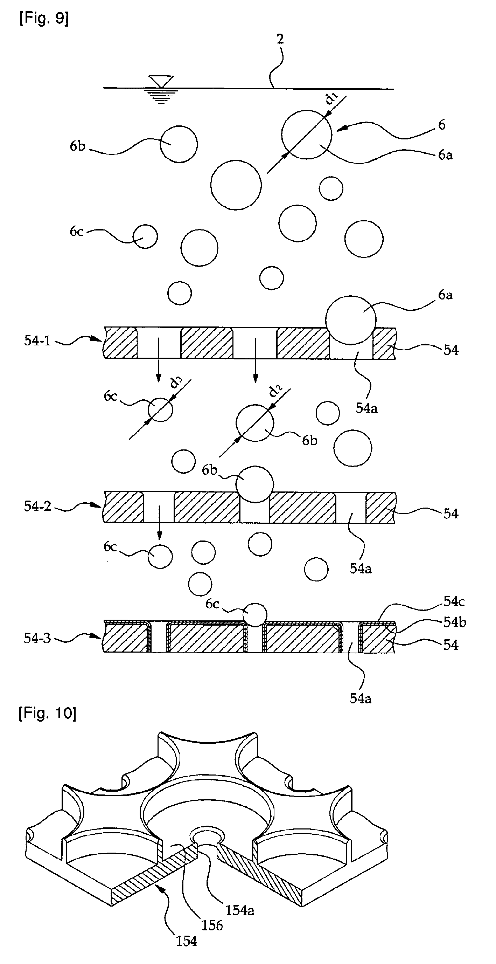

As clearly shown in Figs. 8 and 9, the non-targets 4 have a diameter d smaller than the diameter of the targets 6. For example, the targets 6 include first kind targets 6a, second kind targets 6b and third kind targets 6c. The first kind targets 6a have a first diameter d₁. The second kind targets 6b have a second diameter d₂. The third kind targets 6c have a third diameter d₃. The first diameter d₁ is larger than the second diameter d₂. The second diameter d₂ is larger than the third diameter d₃. The sample 2 may be the physiological fluid such as saliva, sweat or urine, the blood or the serum of a human or an animal. In addition, the fluid containing targets 6 such as cells or tissues of a human, an animal or a plant and the fluid containing viruses or bacteria may be selected as the sample 2. If the blood is selected as the sample 2, the cells of different sizes contained in the blood may become the targets 6. Examples of the cells contained in the blood include red blood cells and white blood cells. In the embodiment of the present invention, the red blood cells may be selected as the non-targets 4.

Referring again to Figs. 1 through 3, the micro-fluidic device according to the present invention includes a filter cabinet 10 forming the outer shell thereof. The filter cabinet 10 includes a body 12 having a first path 14 and a second path 16, both of which are formed within the body 12 so that the sample 2 can flow through the first path 14 and the second path 16. The second path 16 is branched from the first path 14. The first path 14 and the second path 16 are divided by a partition wall 18. A slant surface 18a for guiding the flow of the sample 2 is formed at the upper end of the partition wall 18. The slant surface 18a is inclined downward from the first path 14 toward the second path 16. A third path 20 interconnecting the first path 14 and the second path 16 is formed above the partition wall 18. A fourth path 22 interconnecting the first path 14 and the second path 16 is formed below the partition wall 18.

An introduction hole 24 leading to the upstream end of the first path 14 is formed in the upper portion of the body 12 so that the sample 2 can be supplied through the introduction hole 24. The introduction hole 24 is arranged above the first path 14 so as to supply the sample 2 into the first path 14. A guide 20a for guiding the sample 2 admitted through the introduction hole 24 toward the first path 14 is formed at one side of the third path 20. An discharge hole 26 for discharging the sample 2 is formed in the lower portion of the body 12. The discharge hole 26 is connected to the first path 14 and the second path 16. An open end portion 28 communicating with the first path 14 and the second path 16 is formed on the front surface of the body 12.

A pair of grooves 30 is formed at the upstream end of the first path 14 and is connected to the open end portion 28. The grooves 30 are inclined downward from one side of the first path 14 toward the second path 16. First to fourth grooves 32a through 32d are formed in the second path 16 and are arranged in pair along the flow direction of the sample 2. The first to fourth grooves 32a through 32d are connected to the open end portion 28. Each of the first to fourth grooves 32a through 32d is arranged to extend in a horizontal direction. A cover 34 is fastened to the front surface of the body 12 by screws 36 so as to cover the open end portion 28. The cover 34 may be formed of a door arranged on the front surface of the body 12. The door can be opened and closed by rotating the same about a hinge. A packing may be provided between the body 12 and the cover 34 in order to maintain air-tightness.

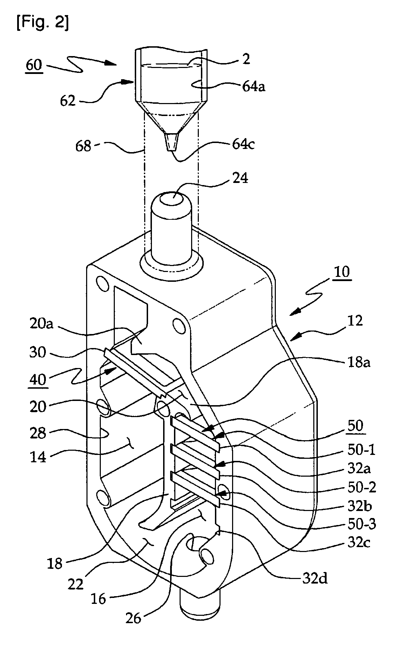

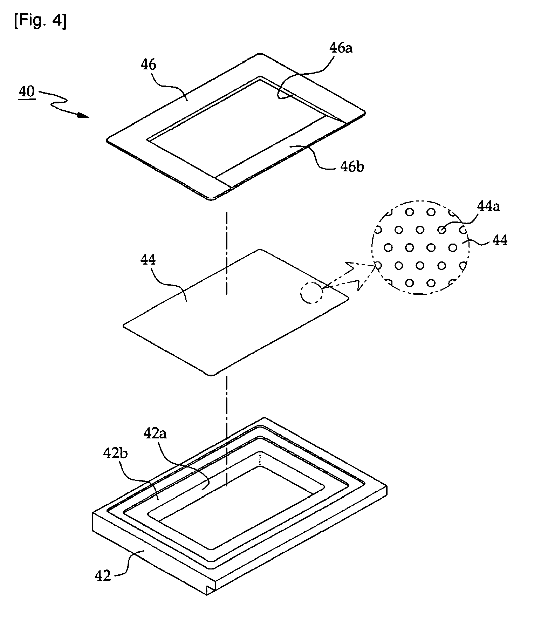

Referring to Figs. 2 through 4 and Fig. 8, the micro-fluidic device according to the present invention includes a first filtering unit 40 obliquely installed in the first path 14 so as to filter the different kinds of targets 6. The first filtering unit 40 includes a support frame 42, a mesh filter 44 and a cover frame 46. The opposite ends of the support frame 42 are fitted to the grooves 30. A hole 42a through which the sample 2 can flow is formed in the central area of the support frame 42. A seat recess 42b is formed on the upper surface of the support frame 42 to extend along the circumference of the hole 42a.

The peripheral edge of the mesh filter 44 is seated on the seat recess 42b. The mesh filter 44 has a plurality of filtering holes 44a formed to filter different kinds of targets 6. The filtering holes 44a are formed to have a diameter smaller than the diameter of the targets 6. The non-targets 4 can pass through the filtering holes 44a but the targets 6 cannot pass through the filtering holes 44a. The mesh filter 44 may be formed to have a thickness of from 10 µm to 50 µm. The filtering holes 44a of the mesh filter 44 can be formed by etching that makes use of a MEMS (Micro-Electro-Mechanical System) technology.

The cover frame 46 is mounted to the seat recess 42b so as to cover the peripheral edge of the mesh filter 44. The peripheral edge of the mesh filter 44 is fixed between the support frame 42 and the cover frame 46. A hole 46a is formed in the central area of the cover frame 46 in alignment of the hole 42a of the support frame 42. A slant surface 46b for guiding the flow of the sample 2 is formed at one side of the hole 46a. The upper surface of the support frame 42 and the upper surface of the cover frame 46 are flush with each other.

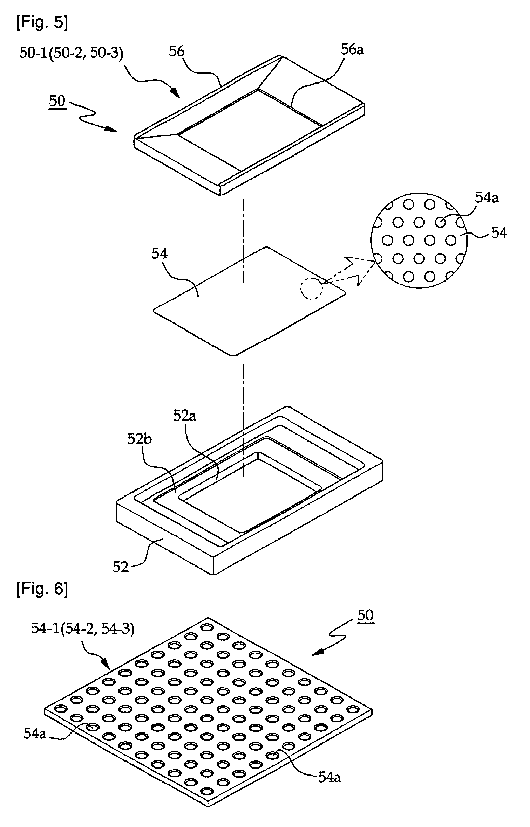

Referring to Figs. 2 and 3, Figs. 5 through 7 and Fig. 9, the micro-fluidic device according to the present invention includes a second filtering unit 50 installed in the second path 16 so that the second filtering unit 50 can receive the different kinds of targets 6 from the first filtering unit 40 and can filter the different kinds of targets 6 on a size-by-size basis. The second filtering unit 50 includes a plurality of filter assemblies 50-1, 50-2 and 50-3 arranged along the flow direction of the sample 2. The filter assemblies 50-1, 50-2 and 50-3 are respectively fitted to the first to third grooves 32a, 32b and 32c in a horizontal posture. Each of the filter assemblies 50-1, 50-2 and 50-3 includes a support frame 52, a mesh filter 54 and a cover frame 56. While no filter assembly is mounted to the fourth grooves 32d in the present embodiment, it may be possible to mount a filter assembly to the fourth grooves 32d.

The opposite ends of the support frame 52 are respectively fitted to each of the first to third grooves 32a, 32b and 32c. A hole 52a through which the sample 2 can flow is formed in the central area of the support frame 52. A seat recess 52b is formed on the upper surface of the support frame 52 to extend along the circumference of the hole 52a. The peripheral edge of the mesh filter 54 is seated on the seat recess 52b. The mesh filter 54 has a plurality of filtering holes 54a formed to filter the different kinds of targets 6. The diameter of the filtering holes 44a may be appropriately set depending on the diameter of the targets 6 so as to have a size suitable for the filtering of the targets 6

The mesh filters 54 of the respective filter assemblies 50-1, 50-2 and 50-3 are arranged such that the diameter of the filtering holes 54a thereof grows smaller along the flow direction of the sample 2. For example, if the filter assemblies 50-1, 50-2 and 50-3 are stacked in three stages, the filtering holes 54a of the first filter assembly 50-1 may be formed to have a diameter of from 15 µm to 20 µm. The filtering holes 54a of the second filter assembly 50-2 may be formed to have a diameter of from 10 µm to 15 µm. The filtering holes 54a of the third filter assembly 50-3 may be formed to have a diameter of from 5 µm to 10 µm.

The cover frame 56 is mounted to the seat recess 52b so as to cover the peripheral edge of the mesh filter 54. The peripheral edge of the mesh filter 54 is fixed between the support frame 52 and the cover frame 56. A hole 56a is formed in the central area of the cover frame 56 in alignment with the hole 54a of the support frame 52. In order to smoothly guide the flow of the sample 2, the hole 56a is formed into a hopper shape so that the cross-sectional area thereof can be gradually reduced from the upstream side toward the downstream side. The upper surface of the support frame 52 and the upper surface of the cover frame 56 are flush with each other.

As shown in Fig. 7, the surface of the mesh filter 54 is coated with a hydrophilic surface layer 54b. The hydrophilic surface layer 54b can be formed by coating the surface of the mesh filter 54 with a hydrophilic material, e.g., titanium oxide (TiO₂) or silicon oxide (SiO₂). The hydrophilic surface layer 54b serves to disperse the flow of the sample 2 making contact with the surface of the mesh filter 54, thereby preventing dew condensation. Likewise, the hydrophilic surface layer 54b may be formed on the surface of the mesh filter 44 of the first filtering unit 40.

An antibody surface layer 54c for capturing cells as the targets 6 is coated on the surface of the hydrophilic surface layer 54b. The antibody surface layer 54c includes an antibody, e.g., an anti-epithelial cell adhesion molecule antibody and an anti-cytokeratin antibody. In the present embodiment, instead of the hydrophilic surface layer 54b, the antibody surface layer 54c may be directly coated on the surface of the mesh filter 54. In Fig. 9, the hydrophilic surface layer 54b and the antibody surface layer 54c are coated on only the mesh filter 54 of the third filter assembly 50-3.

Referring to Figs. 1 through 3, the micro-fluidic device according to the present invention includes a syringe 62 as a sample supply unit 60 for supplying the sample 2 into the introduction hole 24 of the filter cabinet 10. The syringe 62 includes a cylinder 64 having a bore 64a for storing the sample 2, an inlet 64b for introducing the sample 2 and an outlet 64c for discharging the sample 2. The syringe 62 further includes a piston 66 inserted into the bore 64a through the inlet 64b. The piston 66 reciprocates along the bore 64a to discharge the sample 2 through the outlet 64c. The outlet 64c is connected to the introduction hole 24 through a hose 68. The sample supply unit 60 may be formed of a syringe pump or a plunger pump for pumping a specified amount of sample 2 and supplying the sample 2 into the introduction hole 24 of the filter cabinet 10. If the human blood is selected as one example of the sample 2, the sample supply unit 60 may be formed of a blood collection tube, a bag or a pack.

Description will now be made on the operation of the micro-fluidic device of the present invention configured as above.

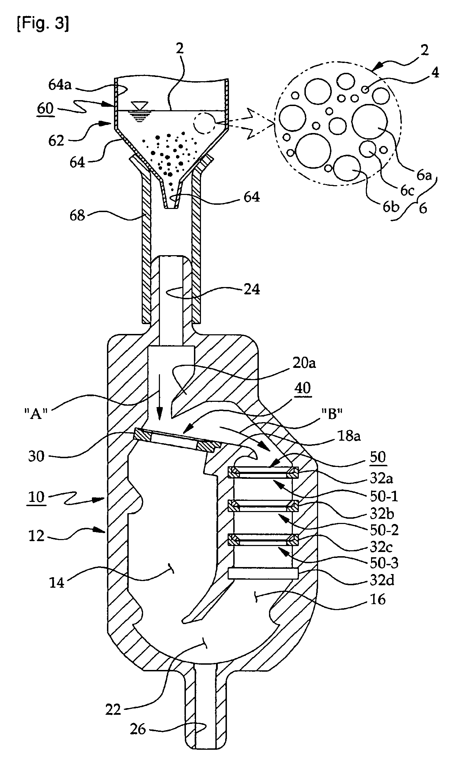

Referring to Figs. 2 and 3, the outlet 64c of the cylinder 64 is connected to the introduction hole 24 of the filter cabinet 10 through the hose 68. If the piston 66 moves forward along the bore 64a of the cylinder 64, the sample 2 is discharged through the outlet 64c. As indicated by an arrow "A" in Fig. 3, the sample 2 flows toward the upstream end of the first path 14 via the hose 68 and the introduction hole 24 of the filter cabinet 10. The sample 2 flowing into the first path 14 passes through the first filtering unit 40 installed in a tilted posture.

Referring to Figs. 3 and 8, the targets 6 of the sample 2 are primarily filtered by pre-treatment performed in the first filtering unit 40. The non-targets 4 of the sample 2 pass through the filtering holes 44a and flow toward the downstream end of the first path 14. The targets 6 cannot pass through the filtering holes 44a. If the filtering holes 44a are formed to have a diameter of 5 µm, the non-targets 4, e.g., the red blood cells having a diameter of from 6 µm to 8 µm, pass through the filtering holes 44a. The red blood cells can pass through a hole having a diameter smaller than the diameter thereof because the cytoplasm surrounding the cell nucleus is deformable. The targets 6, e.g., the cells having a diameter of 5 µm or more, cannot pass through the filtering holes 44a. The targets 6 flow along the surface of the mesh filter 44 and move toward the upstream end of the second path 16 as indicated by an arrow "B" in Fig. 3. At this time, some of the liquid components of the sample 2 pass through the filtering holes 44a and flow toward the downstream end of the first path 14 together with the non-targets 4. The remaining liquid components flow toward the upstream end of the second path 16 along the mesh filter 44. The slant surface 18a of the partition wall 18 and the slant surface 46b of the cover frame 46 serve to smoothly guide the targets 6 flowing from the first filtering unit 40 toward the second path 16.

Referring to Figs. 2, 3 and 9, the different kinds of targets 6 passing through the first filtering unit 40 are secondarily filtered by post-treatment performed in the second filtering unit 50. The different kinds of targets 6 are filtered by the filtering holes 54a of the respective filter assemblies 50-1, 50-2 and 50-3 depending on the size of the targets 6. Among the targets 6, the first kind targets 6a having a size of 15 µm or more cannot pass through the filtering holes 54a of the first filter assembly 50-1. The second kind targets 6b and the third kind targets 6c having a size of less than 15 µm can pass through the filtering holes 54a of the first filter assembly 50-1. Among the targets 6, the second kind targets 6b having a size of 10 µm or more cannot pass through the filtering holes 54a of the second filter assembly 50-2. The third kind targets 6c having a size of less than 10 µm can pass through the filtering holes 54a of the second filter assembly 50-2. The third kind targets 6c having a size of 5 µm or more cannot pass through the filtering holes 54a of the third filter assembly 50-3. The remaining targets having a size of less than 5 µm or the non-targets 4 flowing toward the second filtering unit 50 can pass through the filtering holes 54a of the third filter assembly 50-3. The sample 2 passing through the first filtering unit 40 and the second filtering unit 50 is discharged to the outside of the filter cabinet 10 through the discharge hole 26 and is safely stored in a well-known storage unit such as a tank or a reservoir.

In this manner, the targets 6 are filtered and separated on a size-by-size basis by the filter assemblies 50-1, 50-2 and 50-3 stacked in multiple stages. Accordingly, it is possible to efficiently collect, e.g., the white blood cells of 12 µm to 25 µm in diameter from the human blood. Since the cells are bonded to and captured by the antibody surface layer 54c, it is possible to greatly increase the capture rate of the cells.

Fig. 10 shows a modified example of the mesh filter of the micro-fluidic device according to the present invention. Referring to Fig. 10, the mesh filter 154 of the modified example includes a plurality of pools 156 formed on the upper surface thereof to receive the targets 6. The pools 156 are formed to have a circular cross section. Filtering holes 154a are formed in the central areas of the pools 156 in a concentric relationship with the pools 156. If necessary, the pools 156 may be formed in multiple stages.

Along with the movement of the sample 2, the targets 6 are introduced into the pools 156 and are guided toward the filtering holes 154a. The targets 6a failing to pass through the filtering holes 154a are received in the pools 156. Accordingly, when the sample 2 has been completely filtered, a worker can readily collect the targets 6a received in the pools 156. Since the cells are received in the pools 156, it is possible to prevent deformation of the cells and to increase the filtering rate of the cells.

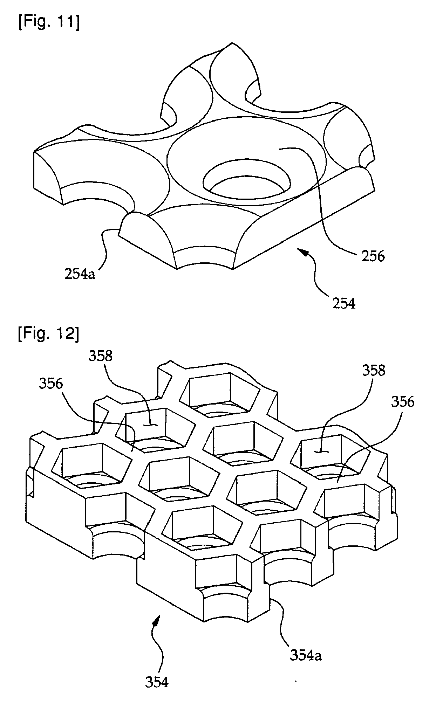

Fig. 11 shows another modified example of the mesh filter of the micro-fluidic device according to the present invention. Referring to Fig. 11, the mesh filter 254 of another modified example includes taper bores 256 connected to the upper end portions of the filtering holes 254a. The taper bores 256 have a diameter gradually decreasing along the flow direction of the sample 2. The taper bores 256 serve to guide the sample 2 toward the filtering holes 254a in order to assure smooth flow of the sample 2. The targets 6a failing to pass through the filtering holes 254a are received in the taper bores 256.

Fig. 12 shows a further modified example of the mesh filter of the micro-fluidic device according to the present invention. Referring to Fig. 12, the mesh filter 354 of the further modified example includes a plurality of guide walls 356 formed on the upper surface thereof to surround the upper edges of the filtering holes 354a so that the guide walls 356 can guide the sample 2 toward the filtering holes 354a. Each of the guide walls 356 includes a bore 358 extending upward from the upper edges of the filtering holes 354a. The guide walls 356 are formed into a honeycomb structure in which the bore 358 has a hexagonal cross section. The guide walls 356 serve to guide the sample 2 so that the sample 2 can be evenly dispersed in the filtering holes 354a.

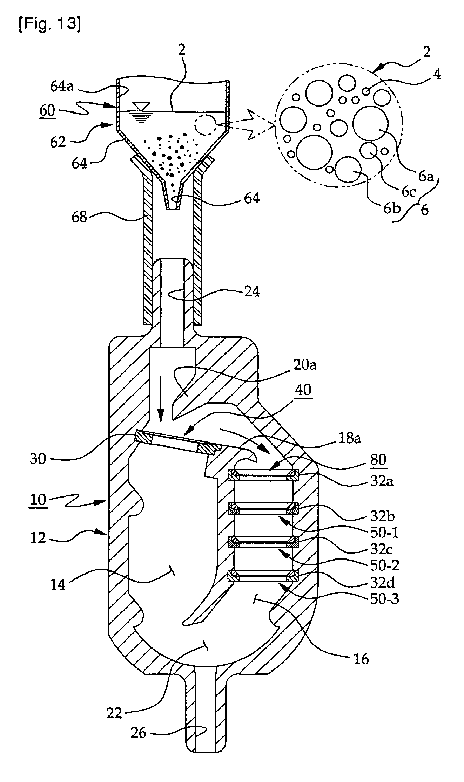

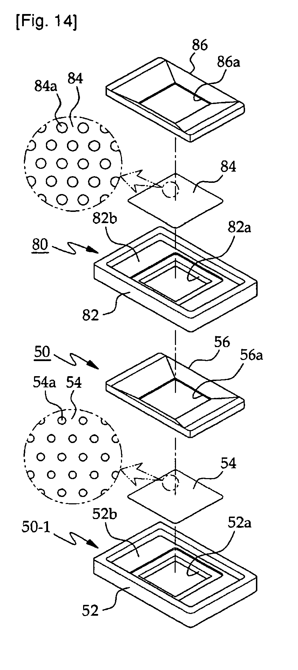

Figs. 13 through 15 show a micro-fluidic device according to another embodiment of the present invention. Referring to Figs. 13 through 15, the micro-fluidic device according to another embodiment includes a dispersing unit 80 installed at the upstream side of the second filtering unit 50 so as to disperse the flow of the sample 2. The dispersing unit 80 is installed in the first grooves 26a of the second path 16. Since the dispersing unit 80 is installed in the first grooves 26a of the second path 16, it is possible to omit one of the filter assemblies 50-1, 50-2 and 50-3. In other words, the dispersing unit 80 may be mounted in place of the first filter assembly 50-1. The second and third filter assemblies 50-2 and 50-3 may be mounted at the downstream side of the dispersing unit 80.

Just like the aforementioned filter assemblies 50-1, 50-2 and 50-3 each having the support frame 52, the mesh filter 54 and the cover frame 56, the dispersing unit 80 includes a support frame 82, a mesh filter 84 and a cover frame 86. The opposite ends of the support frame 82 are fitted to the first grooves 26a. A hole 82a through which the sample 2 can flow is formed in the central area of the support frame 82. A seat recess 82b is formed on the upper surface of the support frame 82 to extend along the circumference of the hole 82a.

The peripheral edge of the mesh filter 84 is seated on the seat recess 82b. The mesh filter 84 includes a plurality of dispersing holes 84a formed so that the different kinds of targets 6 can pass through the dispersing holes 84a. The dispersing holes 84a are formed to have a diameter larger than the diameter of the targets 6. The cover frame 86 is mounted to the seat recess 82b so as to cover the peripheral edge of the mesh filter 84. The peripheral edge of the mesh filter 84 is fixed between the support frame 82 and the cover frame 86. A hole 86a is formed in the central area of the cover frame 86 in alignment with the hole 82a of the support frame 82.

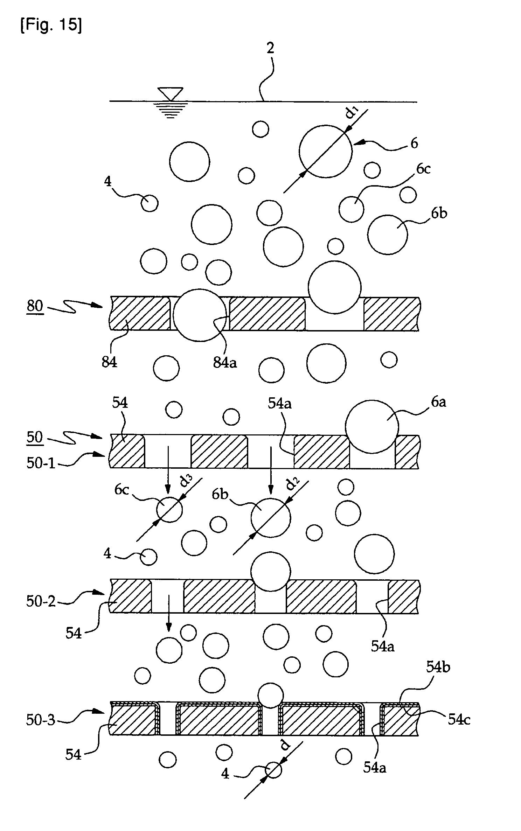

As shown in Fig. 15, the sample 2 is dispersed in the direction orthogonal to the flow direction of the sample 2 when passing through the dispersing unit 80. Along with the flow of the sample 2, the targets 6 contained in the sample 2 are uniformly dispersed in the transverse direction of the second path 16 while passing through the dispersing holes 84a of the mesh filter 84. The human blood as one example of the sample 2 may flow through the second path 16 in a biased state depending on the viscosity thereof. The flow of the blood is dispersed while passing through the dispersing unit 80. This helps increase the filtering rate of the targets 6. The different kinds of targets 6 passing through the dispersing unit 80 are filtered by the second filtering unit 50.

While certain preferred embodiments of the invention have been described above, the scope of the present invention is not limited to these embodiments. It will be apparent to those skilled in the art that various changes, modifications and substitutions may be made without departing from the scope of the invention defined in the claims. Such changes, modifications and substitutions shall be construed to fall within the scope of the present invention.

## Claims

1. A micro-fluidic device, comprising:
a filter cabinet including a first path and a second path branched from the first path, the first path and the second path formed within the filter cabinet so that a sample containing different kinds of targets can flow through the first path and the second path, the filter cabinet further including a introduction hole formed in an upper portion of the filter cabinet to supply the sample into the first path and a discharge hole formed in a lower portion of the filter cabinet to discharge the sample from the first path and the second path;
a first filtering unit installed in an upstream portion of the first path to filter the different kinds of targets from the sample, the first filtering unit configured to guide the different kinds of targets toward the second path; and
a second filtering unit installed in the second path to receive the different kinds of targets from the first filtering unit and to filter the different kinds of targets on a size-by-size basis.

2. The micro-fluidic device of claim 1, wherein the first filtering unit includes:
a support frame mounted to the first path and inclined downward from the first path toward the second path, the support frame having a sample flow hole formed in a central area of the support frame and a seat recess formed on an upper surface of the support frame to extend along a circumference of the sample flow hole;
a mesh filter having a peripheral edge seated on the seat recess and a plurality of filtering holes for filtering the different kinds of targets; and
a cover frame mounted to the seat recess to cover the peripheral edge of the mesh filter, the cover frame having a hole formed in a central area of the cover frame in alignment with the sample flow hole of the support frame.

3. The micro-fluidic device of claim 1, wherein the second filtering unit includes a plurality of filter assemblies mounted to the second path in multiple stages, each of the filter assemblies including:
a support frame mounted to the second path, the support frame having a sample flow hole formed in a central area of the support frame and a seat recess formed on an upper surface of the support frame to extend along a circumference of the sample flow hole;
a mesh filter having a peripheral edge seated on the seat recess and a plurality of filtering holes for filtering the different kinds of targets; and
a cover frame mounted to the seat recess to cover the peripheral edge of the mesh filter, the cover frame having a hole formed in a central area of the cover frame in alignment with the sample flow hole of the support frame.

4. The micro-fluidic device of claim 3, wherein the filter assemblies are arranged such that the diameter the filtering holes is gradually reduced along a flow direction of the sample so as to filter the different kinds of targets on a size-by-size basis.

5. The micro-fluidic device of claim 3, wherein the mesh filter has a surface coated with a hydrophilic surface layer.

6. The micro-fluidic device of claim 5, wherein the sample includes blood containing cells as the different kinds of targets, one of the surface of the mesh filter and the hydrophilic surface layer coated with an antibody surface layer for capturing the cells.

7. The micro-fluidic device of claim 3, wherein the mesh filter includes a plurality of pools formed on an upper surface of the mesh filter to receive the different kinds of targets, each of the filtering holes formed in a central area of each of the pools.

8. The micro-fluidic device of claim 3, wherein the mesh filter includes a plurality of taper bores connected to upper end portions of the filtering holes, the taper bores having a diameter gradually decreasing along a flow direction of the sample.

9. The micro-fluidic device of claim 3, wherein the mesh filter includes a plurality of guide walls formed on an upper surface of the mesh filter to surround upper edges of the filtering holes so that the guide walls can guide the sample toward the filtering holes, each of the guide walls formed into a honeycomb structure having a bore formed on the upper surface of the mesh filter to extend from an edge of each of the filtering holes.

10. The micro-fluidic device of claim 1, further comprising:
a dispersing unit installed at an upstream side of the second filtering unit to disperse the flow of the sample.

11. The micro-fluidic device of claim 10, wherein the dispersing unit includes:
a support frame mounted to the second path, the support frame having a sample flow hole formed in a central area of the support frame and a seat recess formed on an upper surface of the support frame to extend along a circumference of the sample flow hole;
a mesh filter having a peripheral edge seated on the seat recess and a plurality of dispersing holes through which the different kinds of targets can pass; and
a cover frame mounted to the seat recess to cover the peripheral edge of the mesh filter, the cover frame having a hole formed in a central area of the cover frame in alignment with the sample flow hole of the support frame.
